(19) Europäisches Patentamt
European Patent Office
Office européen des brevets

(11) Publication number: **0 165 650**
**B1**

(12) # EUROPEAN PATENT SPECIFICATION

(45) Date of publication of patent specification: **17.05.89**

(51) Int. Cl.$^4$: **C 08 G 65/00**, C 08 G 65/32

(21) Application number: **85300785.4**

(22) Date of filing: **06.02.85**

(54) **Fluoropolyether compounds.**

(30) Priority: **19.06.84 IT 2148184**

(43) Date of publication of application:
**27.12.85 Bulletin 85/52**

(45) Publication of the grant of the patent:
**17.05.89 Bulletin 89/20**

(84) Designated Contracting States:
**BE DE FR GB NL SE**

(56) References cited:
**US-A-4 267 238**
**US-A-4 268 556**

(73) Proprietor: **AUSIMONT S.r.l.**
**31, Foro Buonaparte**
**I-20121 Milano (IT)**

(72) Inventor: **Caporiccio, Gerardo**
**13, Via E. Filiberto**
**I-20149 Milan (IT)**
Inventor: **Strepparola, Ezio**
**6, viale Partigiano**
**I-24047 Treviglio (IT)**
Inventor: **Scarati, Mario Alberto**
**20, via Bronzino**
**I-20133 Milan (IT)**

(74) Representative: **Godwin, Edgar James et al**
**MARKS & CLERK 57-60 Lincoln's Inn Fields**
**London, WC2A 3LS (GB)**

**EP 0 165 650 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to fluoropolyether compounds, and in particular to a new class of compounds having the structure of perfluoropolyether, suitable to be used as lubricants or, in general, as protective agents of surfaces in different applications. The invention more particularly relates to fluoropolyethers containing end groups having an anchoring capacity.

It is known to use perfluoropolyethers as lubricants, for example in the field of video and audio tapes, as well as in precision mechanical instruments subjected to mechanical wear, and in the electrical field, for the protection of contacts subjected to a high number of opening — closing cycles.

Perfluoropolyethers used for such purposes are known commercially under the trade name "Fomblin"® (of Montedison) and Krytox® (of DuPont), and are described for example in US—A—3,242,218, 3,665,041 and 3,715,378.

Improvements in the lubricating properties of the perfluoropolyethers were achieved, according to what is described in US—A—4,268,556 and 4,267,238, by introducing, into the perfluoropolyether chain, end groups of a polar and reactive nature, of the type $—CH_2OH$, —COOR, —CONH—R, and $—CO—CF_3$, which should ensure a better adhesion of the perfluoropolyether compound to the surface to be lubricated.

However, such modification involves the drawback of a high reactivity of the intermediate groups, some of which, for example, easily hydrolyse with formation of acid groups which react with the materials, especially with metallic materials, of the surfaces onto which they are applied, thus altering them.

We have now found a new class of compounds having the structure of perfluoropolyethers, which exhibit better lubricating properties as well as better protective properties than the perfluoropolyethers used so far, and which at the same time are free from the drawbacks described hereinbefore. As a general structure, such compounds consist of a perfluoropolyether chain, characterized by the presence of certain organic end groups.

Such compounds are represented by one of the following general formulas (I) and (II):

(I) $$R—O—(C_3F_6O)_m—(CFXO)_n—CFX—L,$$

in which the groups $(C_3F_6O)$ and (CFXO) are randomly distributed in the chain, and where:

R is one of the groups: $—CF_3$, $—C_2F_5$, $—C_3F_7$;

X is F or $—CF_3$;

m = an integer from 3 to 100;

n = a finite integer, on zero, wherein m + n is from 3 to 100, and preferably from 5 to 50, provided that, when n is a finite integer, the ratio m/n is from 5 to 20.

(II) $$R''CFXO—(C_3F_6O)_x—(CFXO)_y(C_2F_4O)_z—CFX—L,$$

in which the groups $(C_3F_6O)$, (CFXO) and $(C_2F_4O)$ are distributed randomly in the chain, and where:

X is F or $—CF_3$;

R'' is F, $—CF_3$, or $—C_2F_5$, provided that group R''CFX contains not more than 3 carbon atoms, or L, when X is zero;

x = a finite integer, or zero-

y, z = finite integers, such that x + y + z is from 5 to 200, preferably from 5 to 100, provided that, when x is a finite integer, the ratio (x + y)/y is from 5 to 0.5, preferably from 2 to 0.6, while when x is zero, the ratio z/y is from 1 to 0.5 while the sum y + z is from 5 to 200.

When n is a finite integer, R, is preferably $—CF_3$; while when n is zero, R is preferably $—C_2F_5$ or $—C_3F_7$; and when x is zero, X is preferably F.

In general formulas (I) and (II), groups L are organic, non-polymerizable groups, having formula:

(III) A—Y

where:

A = $—CH_2O—$, $—CH_2—O—CH_2—$, $—CF_2—$, or $—CF_2O—$;

Y = an organic non-fluorinated radical, of one of the following two general formulas:

(IV) 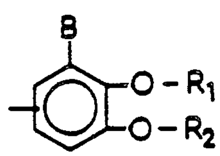

(V) 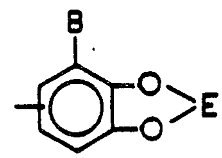

where:

$R_1$, $R_2$ which may be the same as or different from each other, are alkyl radicals containing from 1 to 3 carbon atoms;

E = group $—CHR_3—$ or $—CH_2—CHR_3—$;

$R_3$ = H, or an alkyl radical containing from 1 to 3 carbon atoms;

B = H, or a radical $—OR_3$.

Preferably, radical Y has the formula:

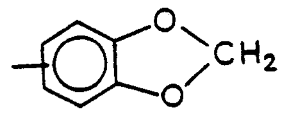

The compounds of formulas (I) and (II), due to group L present therein, have the property of anchoring onto the surfaces of the metals or of the products indicated in groups (a), (b), (c) and (d) mentioned below. Such surfaces may be pure surfaces and directly exposed to the contact of foreign or external agents and bodies, or they may be coated by thin and discrete layers of protective materials, such as resins, amorphous carbon graphite.

Particular examples of surfaces (a) are surfaces of steels, bronzes, brasses, also sintered, surfaces of Sn—Ag, Sn—Au, Sn—Sb alloys, surfaces of alloys such as Co—Cr, Co—Ni, Fe—Co—Ni, Co—Ni—P, deposited by sputtering or electrochemically, or surfaces consisting of metal powders finely dispersed in a polymeric matrix.

Examples of surfaces (b) are surfaces of the materials obtained by spreading suspensions of pigments such as $Fe_2O_3$, $CrO_2$, $Ba(FeO_2)_2$ in polymeric matrices such as polyvinyl chloride, polyvinyl acetate, or thermosetting materials or matrices such as polyurethane or epoxy resins, spread on carriers or supports based on polyester resins, polyamide resins, or on metal carriers or supports such as Al.

Examples of surfaces (c) are surfaces of ceramic materials of the type of synthetic $Al_2O_3$ (sapphire), $CaTiO_3$, $BaTiO_3$, usually utilized in the manufacture of recording and/or reading heads on magnetic means or as supports, particularly of pins, for horology or precision mechanics.

Examples of surfaces (d) are synthetic polymers, also of the elastomeric type, such as polyamides, polyimides, polyetherketones, aromatic polyethersulphones, polysulphones, polycarbonates, polyacetals, and in general the technopolymers utilized for the manufacture of motion members in the mechanical, aeronautical and horological fields, and furthermore the fluorinated, silicone, acrylic, and nitrilic (nitrilacryl) rubbers. In their utilisation as lubricants for such polymers, the products of the present invention may be admixed with the polymers in amounts of from 0.5 to 5% by weight, prior to calendering, banburizing or extrusion operations, or they may be applied onto the surfaces of the polymeric articles ready for use.

The compounds of formula (I) or (II) protect the surfaces coated by them from wear, when such surfaces are subjected to sliding, rolling, tangential or normal forces or stresses, or to any movement susceptible of repeating continuous or discontinuous contact by other surfaces, which may consist of the same materials as listed under (a), (b), and (c). The lubricating action of compounds (I) and (II) is exerted when they are deposited onto the abovesaid materials in continuous layers having thicknesses ranging from a few $\times 10^{-9}$ to a few $\times 10^{-7}$ m (a few tens to a few thousands of Å). Such lubricating action is exerted also on surfaces of synthetic polymers, such as those listed under (d).

In particular the compounds according to the present invention, when deposited onto the surface (a), have the capability of protecting such surfaces from the action of atmospheric agents even in the presence of high humidity.

The compounds of the invention may be used also to promote the residence, on the surfaces of the materials listed under (a), (b), (c), (d), of neutral perfluoropolyether layers provided with a structure of type ($C_1$), ($C_2$), ($C_3$), as specified hereinbelow, which are well known as lubricants having considerable thermal and chemical stabilities.

($C_1$): $R_f—O—(C_3F_6O)_m(CF_2O)_nR'_f$

wherein:

$R_f$ = $—CF_3$, $—C_2F_5$, or $C_3F_7$;

$R'_f$ = an organic radical the same as or different from $R_f$;

m is an integer from 8 to 100;

n may be equal to or different from zero, wherein, if n is zero, $R_f$ is preferably $—C_3F_7$, while $R'_f$ is $—C_2F_5$ or $—C_3F_7$, while if n is different from zero, the m/n is from 5 to 20, while the sum m + n is from 10 to 100;

($C_2$): $R''_f—O—(C_2F_4O)_p(CF_2O)_qR'''_f$

wherein:

$R''_f$ and $R'''_f$, which may be the same as or different from each other, may be $—CF_3$ or $—C_2F_5$, the p/q ratio is from 0.5 to 5, and the sum p + q is from 20 to 200.

($C_3$): $R''_f—O—(C_2F_4O)_r(CF_2O)_5(C_3F_6O)_vR'''_f$

wherein:

$R''_f$ and $R'''_f$, which may be the same as or different from each other, are the same as in the preceding structure, the r/s ratio is from 0.5 to 5, the v/r + s ratio is from 0.01 to 0.4 and the sum v + r + s is from 10 to 300.

Mixtures of the compounds of formula (I) and/or (II) with the neutral oils of types ($C_1$), ($C_2$), ($C_3$) described hereinabove may include from 1% to 50% by weight, but preferably from 1% to 10% by weight, of compounds (I) and/or (II).

In such mixtures, the lubricating action is improved even for small thicknesses of the layer which are from 5—10 × $10^{10}$ to a few × $10^{-7}$ m (5—10 Å to a few thousands of Å), and in sliding or rolling conditions between two surfaces even in very rapid relative motion, up to 5—10 km/minute.

The capability of the abovesaid mixtures of forming thin layers permanently adsorbed on surfaces of type (a), (b) and (c) is determined by vertical spreading measurements performed by immersing a minor side of a rectangular lamina (15 × 100 × 1 mm) made of a material of type (a), (b) or (c) into pans or cups containing the lubricating mixtures and by measuring the thickness of the lubricating film which forms in the course of time at different distances from the free surface of the lubricating liquid in the pan. Such thickness can be determined by ESCA analysis, or by FT—IR spectrophotometry (spectrometry) by reflection on the examined surface.

.The mechanical resistance of the films consisting of the compounds according to the present invention was checked by interposing proportioned amounts of the compounds between two laminas of the metals listed under (a) and connected with an electric circuit in order to measure the resistance opposed by the film to the passage of the electric current: one of the laminas was subjected to a known load (0.5 kg) and to a reciprocating motion with a stroke of 0.5 mm at a known frequency (2—50 Hz). It may be observed that the electrical resistance, which initially was infinite, remains high after many tens of hours of reciprocating motion when between the metal laminas there are interposed compounds (I), (II) or a mixture thereof in an amount of 1—50% with an oil listed under ($C_1$), ($C_2$), ($C_3$). The lubrication of parts in continuous or reciprocating motion, carried out by means of the compounds of types (I) and (II), according to the invention, or by mixtures thereof with the perfluoropolyether oils of aforesaid type C, permits wearing phenomena of sliding, rolling, vibrating parts, or on contacts subjected to repeating opening-closing cycles, to be avoided, thus allowing uniform performances.

Among the technical fields of particular interest in the utilization as lubricants of compounds (I) and (II) according to the present invention, there may be mentioned magnetic means (tapes), electric contactors, ball or roller bearings, and bushes made of metals or sintered alloys, and in such case also the protective action of compounds (I) and (II) against atmospheric agents is very effective.

In particular in the field of magnetic tapes, the surface to be protected from wear caused by contact with a reading head is composed of a film of a magnetic pigment, dispersed in a polymeric matrix, of type (b) described above.

Lubricating films based on compounds (I) and (II) according to the present inventon, or on mixtures thereof with perfluoropolyether oils of type (C), may be applied by immersion, spreading or spraying of the compounds as such or of dilute solutions thereof, for example at 0.5—5% of the lubricating compound or mixture, in 1,1,2-trichlorotrifluoroethane, and by subsequent evaporation of the solvent.

The same lubricant application technique may be adopted when the magnetic material is of the metallic type, i.e. it consists of thin layers of metals such as Co—Cr, Co—Ni, or Co—Ni—P, of the type (a) described above, deposited onto rigid or flexible carriers, by vacuum evaporation, sputtering or electrodeposition techniques.

The lubrication of magnetic means by the compounds of type (I) or (II) or by mixtures thereof with perfluorinated oils of type (C) permits there to be overcome, in the case of metal flexible discs, endurance tests of the order of 3—10 millions of passages on the same path by a reading head which is usually based on synthetic $Al_2O_3$, $BaTiO_3$ or $CaTiO_3$.

The wear resistance of magnetic tapes was measured by subjecting the tape, either or not lubricated with the compounds of the present invention, maintained in reciprocating motion with a stroke of 6.78 cm under the tension of a force of 226.8 g applied over a tape width of 1.27 cm, to the action of a steel ball having a 8 mm diameter and loaded by a 78 g load until passage of light through the carrier surface is observed, after the magnetic layer had been removed by wear.

The compounds of type (I) or (II) and mixtures thereof with perfluorinated oils of type (C) are useful for forming thin protective layers against wear due to sliding or to contact, on electric contactors, where the parts intended for contact for electric current passage are composed of noble metals such as Au, Ag, Pt or of alloys containing Au and Ag.

In this case also, the lubricating film is deposited by immersion or spraying with a solution of the lubricant in 1,1,2-trichlorotrifluoroethane.

In the case of electromagnetic contactors with lamellar-type nuclei, the compounds of type (I) or (II) and mixtures thereof with compounds of type (C) efficaciously damp the vibrations of the contractor.

The compounds of the present invention are useful as lubricants of manufactured articles based on technopolymers subjected to movement and to contact with other surfaces.

Examples of such manufactured articles are gears, porous bushes, sliders, contacts, and components of ball or roller bearings.

The compounds of the present invention may also be usefully included in the formulation of greases consisting of suspending liquids of apolar nature, and of thickeners consisting of organic polymeric materials or of inorganic materials.

The effect of the compounds of type (I) and (II) is that of enhancing the mechanical stability of greases, of reducing oil leakage during work, and of increasing anti-wear effect. Examples of greases which can be improved by the compounds of the present invention, used in amounts of from 0.1 to 2%, are greases formulated with mineral, silicone or perfluoropolyether oils and thickened with PTFE, TFE—EFP (FEP)

copolymers, silica, graphite, $MoS_2$, and bentonites.

The compounds of formula (I) and (II) of the present invention, can be prepared starting from known compounds having respectively the following structures:

$$(D_1): \quad R{-}O{-}(C_3F_6O)_m{-}(CFXO)_n{-}CFX{-}K,$$

$$(D_2): \quad R''CFXO{-}(C_3F_6O)_x{-}(CFXO)_y{-}(C_2F_4O)_z{-}CFX{-}K,$$

wherein:

R, R'', X, m, n, x, y, z are the same as defined hereinbefore, and R'' may also be the same as K, and where K is a functional group of the type

—COOM (M = H, $CH_3$),

—CHO,

—$CH_2OH$,

—COCl,

—COF,

by reaction with compounds of formula D—Y, where Y has the meaning previously specified and D is a functional group capable of reacting with group K to yield A.

Compounds in which A = —$CH_2O$— are obtained from precursors of formula $D_1$ or $D_2$ in which K = —$CH_2OH$, by condensation in an alkaline medium with products containing group YCl with displaceable halogen.

Compounds in which A = —$CH_2OCH_2$— are usually obtained from hydroxymethyl precursors of formula $D_1$ or $D_2$ by alkaline condensation with compounds containing a chloro- or bromo-methylated aromatic ring in group Y.

Compounds in which A = —$CF_2O$— are obtained from precursors $D_1$ or $D_2$ in which K = ether group (obtained from compounds $D_1$ or $D_2$ where K = —COOH, or —COO-alkyl, or also —COCl, by reaction with compounds of formula Y—OH) by fluorination of group C=O to —$CF_2$— with $HF/SF_4$ mixtures, at temperatures from 120°C to 180°C and at pressures from 20 to 60 atm., with reaction times of 20—25 hours, following under I.R. the disappearance of the absorption due to group CO.

Compounds where A = —$CF_2$— are obtained by reaction of precursors $D_1$ or $D_2$, in which K = —COCl or —COF, with products of formula DY, wherein D is a metal of the type Li, Mg, Cu, under known conditions for obtaining ketone compounds of formula $D_1$ —CO—Y or $D_2$—CO—Y, carrying out the conversion of group C=O to —$CF_2$ with $HF/SF_4$ mixtures, at temperatures from 120°C to 180°C and at pressures from 20 to 60 atm., with reaction times of 20—25 hours, following under I.R. the disappearance of the absorption due to group =CO.

The invention will be further described with reference to the following illustrative examples.

## Example 1

75 g of a diol of formula

$$HOCH_2{-}CF_2O{-}(C_2F_4O)_m{-}(CF_2O)_p{-}CF_2{-}CH_2{-}OH$$

(where m + p = 25, m/p = 0.6) having a M.W. = 2300, were added to a solution of 8 g of potassium tert-butylate in 150 ml of tert-butyl alcohol maintained at 30°C.

Stirring was carried on for 2 hours. Successively, 13 g of 4-chloromethyl-1,2-methylendioxybenzene were added. Precipitation of potassium chloride was immediately observed. Reaction was continued during 3 hours, always at 30°C, and then the reaction mixture was poured into water containing 0.4% of hydrochloric acid.

The layers then separated; the heaviest one, after drying under the vacuum created by a mechanical pump at 100°C was filtered on a membrane. Thus, there were obtained 76 g of limpid liquid which, on I.R. and N.M.R. analyses, was found to consist of a compound having the following structure:

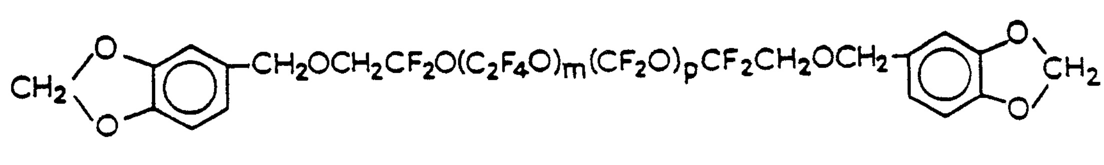

A magnetic tape with $CrO_2$ pigment was spread on a reverse roll coater with a 1% solution of such compound in 1,1,2-trichlorotrifluoroethane. A specimen of a tape so treated was subjected to the abrasion action of a steel ball having a 0.32 diameter, loaded with a 28 g load. The ball was subjected to a reciprocating motion along a 6.78 cm run. The tape was under a tension caused by a force equal to 226.8 g applied onto a tape width equal to 1.27 cm.

After a time corresponding to 8000 passages, no signs of abrasion were noticed, while after 350 passages the non-lubricated tape exhibited signs of abrasion revealed by the passage of light.

The product of this example was also utilized in the lubrication of metallized floppy disks prepared by sputtering of two layers (lower layer made of permalloy and upper layer made of Co—Cr).

The coating with lubricant was carried out by dip coating in a 1% solution of the compound in 1,1,2-trichlorotrifluoroethane. At an extraction speed of 5 mm/sec the resulting thickness was $5 \times 10^{-3}$m (500Å). After rotation in a liner, the actual thicknesses were about $2 \times 10^{-3}$m (200Å). The discs so lubricated were subjected to wearing tests according to standard ANSI through rotation on the same path until a limit of 3 million revolutions.

Under a 15 g load of the head, the disc resisted during 4.2 million revolutions before showing signs of wear detectable by optical microscopy. A subsequent examination with an E.D.S. microfeeler proved that the wear was only superficial and had not affected the Co—Cr layer until bringing to surface the permalloy the under-layer.

A 2% solution of compound (b) in 1,1,2-trichlorotrifluoroethane was utilized to lubricate the surface of a video tape prepared by depositing (by means of the high incidence deposition technique) a layer of Co—Ni alloy having a thickness of 0.2 µ onto a polyester resin film having a thickness of 12 µ.

Two tape samples, one of which was lubricated and the other not lubricated, were subjected to comparative friction coefficient and still frame life tests.

The (dynamic) friction coefficient measured between non-lubricated tape and a ceramic slider was found to be equal to 0.55. The same determination on the tape lubricated by the product in question led to a friction coefficient of 0.2.

Still frame tests showed that the non-lubricated tape was not capable of withstanding the still frame run for more than 10 seconds, while the lubricated tape was able to run during 5 minutes under the same conditions without serious damage and with an absolutely acceptable drop-out rate.

Example 2

45 g of a compound of formula

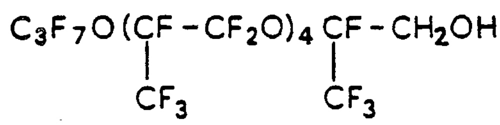

having a M.W. = 980, were added to a solution of 5.5 g of potassium tert.-butylate in 100 ml of tert.-butyl alcohol, maintained at 30°C. After a 2-hour reaction, 5.5 g of 4-chloromethyl-1,2-methylenedioxybenzene were added, the whole was stirred during 3 hours at 30°C, whereafter the mixture was poured into 200 ml of an aqueous solution containing 0.4% of hydrochloric acid. The heaviest layer formed was then separated, which after concentration by evaporation at 100°C under vacuum, was filtered on a membrane. 43 g of a limpid liquid were thus obtained which, on I.R. and N.M.R. analyses, was found to consist of a compound having the following structure:

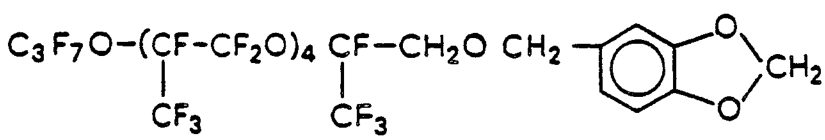

A 1% solution of such compound in 1,1,2-trichlorotrifluoroethane was used to coat floppy disks of the same type as described in example 1. The disks, treated in like manner as described in example 1 and subjected to wearing tests according to ANSI standards, revealed wear signs under an optical microscope after 3.5 million rotations on the same path.

A mild steel lamina measuring 3 × 50 × 100 mm, polished and cleaned of oily residues, if any, was immersed into the product obtained as described hereinbefore and was extracted at a speed of 10 mm/sec. It was allowed to stand for 12 hours, whereafter it was kept during 28 hours in a non-saline mist chamber at 50°C and at 100% of relative humidity.

At the end of the test, the lamina did not exhibit rust traces.

For comparison purposes, a lamina treated in the same manner in a fluoroether oil, "Formblin" type, having a viscosity of $250 \times 10^{-6} m^2/s$ (250 Cst) at 20°C, exhibited evident rust signs at the end of the test. "Formblin" is a Registered Trade Mark.

**Claims**

1. Fluoropolyether compounds, having the general formula

(I) $R—O—(C_3F_6O)_m—(CFXO)_n—CFX—L$, or

(II) $R''CFXO—(C_3F_6O)_x—(CFXO)_y—(C_2F_4O)_z—CFX—L$,

where

$R = -CF_3, -C_2F_5$, or $-C_3F_7$;

$X = F$, or $-CF_3$;

$R'' = F, -CF_3$, or $-C_2F_5$, provided that group $R''CFX$ contains not more than 3 carbon atoms, or L when x is zero;

m = an integer from 3 to 100;

n = a finite integer, or zero, wherein $m + n$ is from 3 to 100, provided that, when n is a finite integer, m/n is from 5 to 20;

x = a finite integer, or zero;

y, z = finite integers, such that $x + y + z$ is from 5 to 200, provided that, when x is a finite integer, $(x + z)/y$ is from 5 to 0.5, while when x is zero, z/y is from 1 to 0.5 and $y + z$ is from 5 to 200;

L = a group A—Y where

$A = -CH_2O-, -CH_2-O-CH_2-, -CF_2-$, or $-CF_2O-$;

Y = an organic radical of one of the following formulas

(IV) 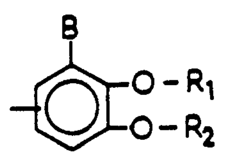

(V) 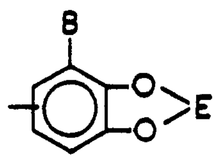

where:

$R_1, R_2$ = alkyls $C_1-C_3$,

$E = -CHR_3-$ or $-CH_2-CHR_3-$,

$R_3$ = H or an alkyl $C_1-C_3$,

B = H or a radical $-OR_3$.

2. A compound as claimed in claim 1, wherein R is $-CF_3$ when n is a finite integer, R is $-C_2F_5$ or $-C_3F_7$ when n is zero, and X is F when x is zero.

3. A compound as claimed in claim 1 or 2, wherein the radical Y has the formula

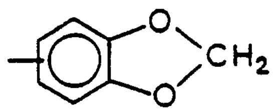

4. The use of the compounds according to any of claims 1, 2 and 3, as coatings of metallic, ceramic, or polymeric material surfaces, or of surfaces obtained by filming or spreading of suspensions of inorganic and/or organic pigments in polymeric matrices.

5. A mixture characterized by indicating 1—50% by weight of at least one compound as claimed in claim 1, 2 or 3, and at least one perfluoropolyether of the formulas

$(C_1)$: $R_f-O-(C_3F_6O)_m(CF_2O)_nR'_f$

wherein:

$R_f = -CF_3, -C_2F_5$, or $C_3F_7$;

$R'_f$ = an organic radical the same as or different from $R_f$;

m is an integer from 8 to 100;

n may be equal to or different from zero, wherein, if n is zero, $R_f$ is preferably $-C_3F_7$, while $R'_f$ is $-C_2F_5$ or $-C_3F_7$, while if n is different from zero, the m/n is from 5 to 20, while the sum $m + n$ is from 10 to 100;

$(C_2)$: $-O-(C_2F_4O)_p(CF_2O)_qR'''_f$

wherein:

$R''_f$ and $R'''_f$, which may be the same as or different from each other, may be $-CF_3$ or $-C_2F_5$, the p/q ratio is from 0.5 to 5, and the sum $p + q$ is from 20 to 200.

$(C_3)$: $R''_f-O-(C_2F_4O)_r(CF_2O)_s(C_3F_6O)_vR'''_f$

wherein:

$R''_f$ and $R'''_f$, which may be the same as or different from each other, are the same as in the preceding structure, the r/s ratio is from 0.5 to 5, the $v/r + s$ ratio is from 0.01 to 0.4 and the sum $v + r + s$ is from 10 to 300.

6. The use of the mixtures according to claim 5, as coatings of metallic, ceramic, or polymeric material surfaces, or of surfaces obtained by filming or spreading of suspensions of inorganic and/or organic pigments in polymeric matrices.

7. Magnetic recording means, being provided with a coating consisting of or including at least one compound as claimed in claim 1, 2 or 3.

8. Magnetic recording means, being provided with a coating consisting of or including a mixture as claimed in claim 5.

9. A process for preparing a compound according to claim 1, characterized by reacting a compound having the formula:

($D_1$): $R—O—(C_3F_6O)_m—(CFXO)_n—CFX—K$, or

($D_2$): $R''CFXO—(C_3F_6O)_x—(CFXO)_y—(C_2F_4O)_z—CFX—K$,

where R, R'', X, m, n, x, y, z are as defined as in claim 1, and R'' may be the same as K, where K is a functional group of the type
—COOM (M = H, $CH_3$),
—CHO
—$CH_2OH$,
—COCl,
—COF,
with a compound of the formula D—Y, where Y is as defined in claim 1 and D is a functional group capable of reacting with group K to yield A.

**Patentansprüche**

1. Fluorpolyether-Verbindungen mit der allgemeinen Formel

(I) $R—O—(C_3F_6O)_m—(CFXO)_n—CFX—L$ oder

(II) $R''CFXO—(C_3F_6O)_x—(CFXO)_y—(C_2F_4O)_z—CFX—L$,

in welchen
R = $—CF_3$, $—C_2F_5$ oder $—C_3F_7$;
X = F oder $—CF_3$;
R'' = F, $—CF_3$ oder $—C_2F_5$, mit der Maßgabe, daß die Gruppe R''CFX nicht mehr als 3 Kohlenstoffatome enthält, oder L, wenn x 0 ist;
m = eine ganze Zahl von 3 bis 100;
n = eine endliche ganze Zahl oder 0, wobei m + n von 3 bis 100 beträgt, mit der Maßgabe, daß wenn n eine endliche ganze Zahl ist, m/n 5 bis 20 ist;
x = eine endliche ganze Zahl oder 0;
y, z = endliche ganze Zahlen, so daß x + y + z 5 bis 200 beträgt, mit der Maßgabe, daß wenn x eine endliche ganze Zahl ist, (x + z)/y 5 bis 0,5 beträgt, während wenn x 0 ist, z/y 1 bis 0,5 und y + z 5 bis 200 ist;
L = eine Gruppe A—Y, in welcher
A = $—CH_2O—$, $—CH_2—O—CH_2—$, $—CF_2—$, oder $—CF_2O—$;
Y = ein organischer Rest mit einer der folgenden Formeln

(IV) 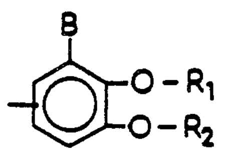 (V) 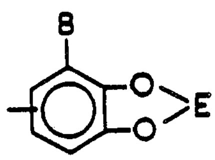

in welchen
$R_1$, $R_2$ = $C_1$—$C_3$-Alkylgruppen,
E = $—CHR_3—$ oder $—CH_2—CHR_3—$,
$R_3$ = H oder $C_1$—$C_3$-Alkylgruppen,
B = H oder ein Rest $—OR_3$.

2. Verbindung nach Anspruch 1, in welcher R für $—CF_3$ steht, wenn n eine endliche ganze Zahl ist, R $—C_2F_5$ oder $—C_3F_7$ bedeutet, wenn n 0 ist und X für F steht, wenn x 0 ist.

3. Verbindung nach Anspruch 1 oder 2, in welcher der Rest Y die Formel

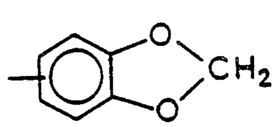

hat.

4. Verwendung der Verbindungen gemäß irgendeinem der Ansprüche 1, 2 und 3 als Überzüge von metallischen, keramischen oder Polymermaterial-Oberflächen oder von Oberflächen, die erhalten werden durch Filmbildung oder Verteilung von Suspensionen von anorganischen und/oder organischen Pigmenten in polymeren Matrizen.

5. Mischung, dadurch gekennzeichnet, daß sie einschließt 1 bis 50 Gewichtsprozent wenigstens einer Verbindung nach Anspruch 1, 2 oder 3 und wenigstens einen Perfluorpolyether der Formeln

($C_1$): $R_f$—O—$(C_3F_6O)_m(CF_2O)_nR'_f$

in welcher:

$R_f$ = —$CF_3$, —$C_2F_5$ oder $C_3F_7$;

$R'_f$ = ein organischer Rest, der gleich oder verschieden von $R_f$ ist;

m eine ganze Zahl von 8 bis 100 darstellt;

n gleich oder verschieden von 0 sein kann, wobei, falls n 0 ist, $R_f$ vorzugsweise für —$C_3F_7$ steht, während $R'_f$ —$C_2F_5$ oder —$C_3F_7$ darstellt, während, wenn n von 0 verschieden ist, das Verhältnis m/n 5 bis 20 beträgt, wobei die Summe m + n 10 bis 100 ist;

($C_2$): $R''_f$—O—$(C_2F_4O)_p(CF_2O)_qR'''_f$

in welcher:

$R''_f$ und $R'''_f$, die gleich oder verschieden sein können, —$CF_3$ oder —$C_2F_5$ bedeuten können, das Verhältnis p/q 0,5 bis 5 beträgt und die Summe von p + q 20 bis 200 ist;

($C_3$): $R''_f$—O—$(C_2F_4O)_r(CF_2O)_s(C_3F_6O)_vR'''_f$

in welcher:

$R''_f$ und $R'''_f$, die gleich oder verschieden sein können, dieselbe Bedeutung wie in der vorangehenden Struktur haben, das Verhältnis r/s von 0,5 bis 5 beträgt, das Verhältnis v/r + s 0,01 bis 0,4 ist und die Summe v + r + s 10 bis 300 beträgt.

6. Verwendung der Mischungen nach Anspruch 5 als Überzüge von metallischen, keramischen oder Polymermaterial-Oberflächen oder von Oberflächen, die erhalten wurden durch Filmbildung oder Verteilung von Suspensionen von anorganischen und/oder organischen Pigmenten in Polymermatrizen.

7. Magnetisches Aufzeichnungsmittel, versehen mit einem Überzug, der besteht aus oder einschließt wenigstens eine Verbindung nach Anspruch 1, 2 oder 3.

8. Magnetisches Aufzeichnungsmittel, versehen mit einem Überzug, der besteht aus oder einschließt eine Mischung nach Anspruch 5.

9. Verfahren zur Herstellung einer Verbindung nach Anspruch 1, dadurch gekennzeichnet, daß eine Verbindung mit der Formel:

($D_1$): R—O—$(C_3F_6O)_m$—$(CFXO)_n$—CFX—K oder

($D_2$): R''CFXO—$(C_3F_6O)_x$—$(CFXO)_y$—$(C_2F_4O)_z$—CFX—K,

in welcher R, R'', X, m, n, x, y, z wie in Anspruch 1 definiert sind und R'' dieselbe Bedeutung wie K haben kann, wobei K eine funktionelle Gruppe des Typs

—COOM (M = H, $CH_3$),

—CHO

—$CH_2OH$,

—COCl,

—COF,

ist, mit einer Verbindung der Formel D—Y, in welcher Y wie in Anspruch 1 definiert ist und D eine funktionelle Gruppe darstellt, die mit einer Gruppe K reagieren kann, um A zu liefern, umsetzt.

**Revendications**

1. Fluoropolyethers présentant la formule générale:

(I) R—O—$(C_3F_6O)_m$—$(CFXO)_n$—CFX—L ou

(II) R''CFXO—$(C_3F_6O)_x$—$(CFXO)_y$—$(C_2F_4O)_z$—CFX—L,

dans laquelle

R = —$CF_3$, —$C_2F_5$ ou —$C_3F_7$;

X = F ou —$CF_3$;

R'' = F —$CF_3$, ou —$C_2F_5$ lorsque le groupe R''CFX ne contient pas plus de 3 atomes de carbone ou L lorsque x est égal à zéro:

m = un nombre entier compris entre 3 et 100;

n = un nombre entier ou fini ou zéro, la somme m + n étant comprise entre 3 et 100, pourvu que lorsque n est un numbre entier fini, le rapport m/n est compris entre 5 et 20;

x = un nombre entier fini ou zéro

y, z sont des nombres entiers finis tels que la somme x + y + z soit comprise entre 5 et 200 pourvu que

lorsque x est un nombre entier fini, le rapport (x + y)/y soit compris entre 5 et 0,5 alors que lorsque x = zéro, le rapport z/y soit compris entre 1 et 0,5 et la somme y/z soit comprise entre 5 et 200;

L = un groupe A—Y dans lequel:

A = $—CH_2O—$, $—CH_2—O—CH_2—$, $—CF_2—$, ou $—CF_2O—$;

Y = un radical organique présentant l'une ou l'autre des formules

(IV) 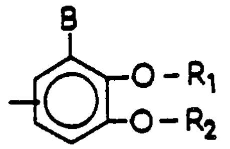 (V) 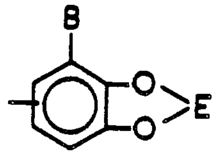

dans lesquelles:

$R_1$, $R_2$ = alkyles en $C_1—R_3$

E = $—CHR_3—$ ou $—CH_2—CHR_3—$

$R_3$ = H ou un alkyle en $C_1—C_3$

B = H ou un radical $—OR_3$

2. Un composé tel que revendiqué dans la revendication 1, caractérisé en ce que R = $—CF_3$ lorsque n est un nombre entier fini, R est $—C_2F_5$ ou $—C_3F_7$ lorsque n = zéro et X est F lorsque x = zéro.

3. Un composé tel que revendiqué dans les revendications 1 ou 2, caractérisé en ce que le radical Y présente la formule

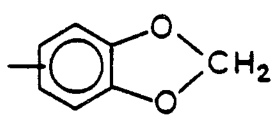

4. L'utilisation des composés selon l'une quelconque des revendications 1, 2 et 3 pour le revêtement de surfaces de matières polymères céramiques ou métalliques ou de surfaces obtenues par étirage sous forme de pellicules ou étalement de suspensions de pigments organiques et/ou minéraux dans des supports polymères.

5. Un mélange caractérisé en ce qu'il inclut 1 à 50% en poids d'au moins un composé tel que revendiqué dans les revendications 1, 2 ou 3 et d'au moins un des perfluoropolyethers présentant des formules suivantes:

($C_1$): $R_f—O—(C_3F_6O)_m(CF_2O)_nR'_f$

dans laquelle:

$R_f$ = $—CF_3$, $—C_2F_5$, ou $C_3F_7$;

$R'_f$ = un radical organique identique ou différent de $R_f$

m = un nombre entier compris entre 8 et 100

n = peut être égal ou différent de zéro, étant entendu que si n = 0, $R_f$ est de préférence $—C_3F_7$, alors que $R'_f$ est $—C_2f_5$ ou $—C_2f_7$, tandis que si n est différent de zéro, le rapport m/n est compris entre 5 et 20 alors que la somme m + n est comprise entre 10 et 100;

($C_2$): $R''_f—O—(C_2F_4O)_p(CF_2O)_qR'''_f$

dans laquelle:

$R''_f$ et $R'''_f$ qui peuvent être identiques ou différents l'un de l'autre peuvent être $—CF_3$ ou $—C_2F_5$, le rapport p/q étant compris entre 0,5 et 5 et la somme p + q entre 20 et 200;

($C_3$): $R''_f—O—(C_2F_4O)_r(CF_2O)_5(C_3F_6O)_vR'''_f$

dans laquelle:

$R''_f$ et $R'''_f$ qui peuvent être identiques ou différents l'un de l'autre sont tels que précités, le rapport r/s étant compris entre 0,5 et 5 et le rapport v/(r + s) étant compris entre 0,01 et 0,4 tandis que la somme v + r + s est comprise entre 10 et 300.

6. L'utilisation des mélanges selon la revendication 5, pour le revêtement de surfaces de matières métalliques, céramiques ou polymères ou de surfaces obtenues par étirage sous forme de pellicules ou étalement de suspensions de pigments organiques ou minéraux dans des supports polymères.

7. Moyens d'enregistrement magnétiques présentant un revêtement formé de ou contenant au moins un composé ainsi que revendiqué dans les revendications 1, 2 ou 3.

8. Moyens d'enregistrement magnétique présentant un revêtement formé de ou contenant un mélange tel que revendiqué dans la revendication 5.

9. Un procédé pour préparer un composé selon la revendication 1, caractérisé en ce qu'il consiste à faire réagir un composé présentant la formule:

($D_1$): $R—O—(C_3F_6O)_m—(CFXO)_n—CFX—K$, ou

$(D_2)$: $R''CFXO—(C_3F_6O)_x—(CFXO)_y—(C_2F_4O)_z—CFX—K$,

dans laquelle:

R, R'', X, m, n, x, y, z sont tels que définis dans la revendication 1 et R' peut être identique à K, K étant un groupe fonctionnel du type

—COOM ($M = H, CH_3$),

—CHO

—$CH_2OH$,

—COCl,

—COF,

avec un composé de formule D—Y, dans lequel Y est tel que défini dans la revendication 1 et D est un groupe fonctionnel susceptible de réagir avec un groupe K pour former A.